# EUROPEAN PATENT SPECIFICATION

(11) **EP 3 373 808 B1**
(45) Date of publication and mention of the grant of the patent: **06.05.2020**
(21) Application number: 16793773.9
(22) Date of filing: 20.10.2016
(51) Int. Cl.: A61B 5/05, G01N 27/22, G01R 29/08, H01Q 9/26, A61B 5/00

(54) **QUANTIFICATION OF INHOMOGENEITIES IN OBJECTS BY ELECTROMAGNETIC FIELDS**
QUANTIFIZIERUNG VON INHOMOGENITÄTEN BEI OBJEKTEN MITTELS ELEKTROMAGNETISCHER FELDER
QUANTIFICATION D'INHOMOGÉNÉITÉS DANS DES OBJETS PAR DES CHAMPS MAGNÉTIQUES

(30) Priority: 09.11.2015 SE 1530175; 14.02.2016 SE 1630027
(43) Date of publication of application: 19.09.2018
(73) Proprietor: Scanwaves AB, 685 25 Torsby (SE)
(72) Inventor: OTTERSKOG, Magnus, 732 97 Arboga (SE); PETROVIC, Nicola, 732 97 Arboga (SE); RISMAN, P O, 732 97 Arboga (SE)
(74) Representative: AWA Sweden AB
(86) International application number: PCT/EP2016/075181
(87) International publication number: WO 2017/080775

(56) References cited:
- JP-A- 2007 278 820
- JP-A- 2013 044 660
- US-A- 5 841 288
- US-A- 6 160 525
- US-A1- 2015 103 859
- PETROVIC N ET AL: "Antenna applicator design for microwave imaging of the interior of human breasts", JOURNAL OF PHYSICS D: APPLIED PHYSICS, INSTITUTE OF PHYSICS PUBLISHING LTD, GB, vol. 47, no. 38, 28 August 2014 (2014-08-28), page 385401, XP020270199, ISSN: 0022-3727, DOI: 10.1088/0022-3727/47/38/385401 [retrieved on 2014-08-28]
- MOBASHSHER AHMED TOAHA ET AL: "Microwave System to Detect Traumatic Brain Injuries Using Compact Unidirectional Antenna and Wideband Transceiver With Verification on Realistic Head Phantom", IEEE TRANSACTIONS ON MICROWAVE THEORY AND TECHNIQUES, IEEE SERVICE CENTER, PISCATAWAY, NJ, US, vol. 62, no. 9, 1 September 2014 (2014-09-01), pages 1826-1836, XP011557627, ISSN: 0018-9480, DOI: 10.1109/TMTT.2014.2342669 [retrieved on 2014-08-29]
- TWIG YGAL ET AL: "Sensitive surface loop-gap microresonators for electron spin resonance", REVIEW OF SCIENTIFIC INSTRUMENTS, AIP, MELVILLE, NY, US, vol. 81, no. 10, 21 October 2010 (2010-10-21), pages 104703-104703, XP012145495, ISSN: 0034-6748, DOI: 10.1063/1.3488365

## Description

### Field of the invention

The present invention relates to quantification of inhomogeneities in objects by means of electromagnetic fields. In particular, the present invention relates to a system for direct detection of dielectric irregularities (deviations of electromagnetic properties) inside an object under study. Embodiments of the invention are suitable for investigations of tissue irregularities caused by for example tumors, necroses or hemorrhages in human breasts or heads. Alternative embodiments may be suitable for other medical or industrial applications.

### Technical background

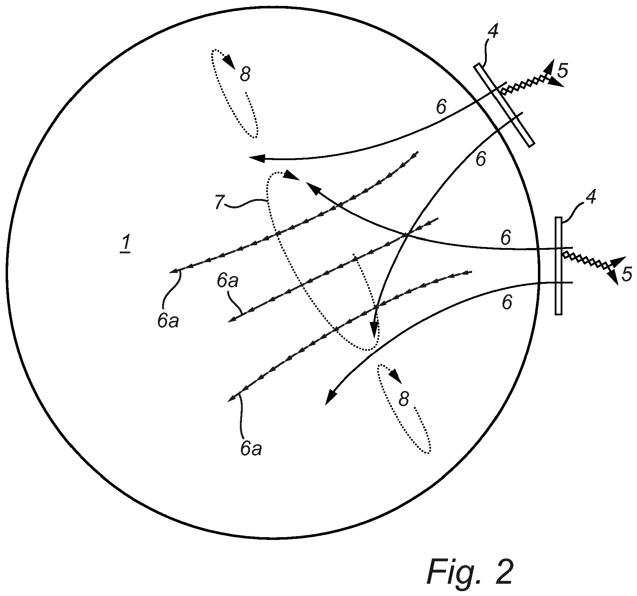

There are several kinds of prior art antennas described in the literature for the general purposes above. These are then designed for microwaves, typically for frequencies between about 1 GHz and 6 GHz. Examples are:
1. Coaxial endfire applicator. - A typical example is described in US patent 6,287,302. Their field matching is therefore poor and only a thin zone of the object under study (OUS) nearby the applicator will be significantly influenced.
2. Circular TM₀₁ mode endfire applicator. - This is described in US patent 4,392,039. A high permittivity dielectric filling is necessary for both achieving a suitably small applicator diameter when fed by an inexpensive ISM 2450 MHz band microwave generator, and for achieving a desirable so-called magnetic wall effect at resonance resulting in the resonant standing wave in the applicator having a dominant electric field parallel to the OUS interface; see Figure 2 in the referenced patent. The permittivity of the low loss dielectric filling of the applicator is thus significantly higher than that of the OUS, so titanium dioxide (ε' = 90) is preferred. The energy penetration depth in the OUS then typically becomes flat ellipsoidal, with a depth away from the blunt end of the applicator of up to about five millimeters.
3. Other circular endfire applicators using a rotationally symmetric microwave mode of the kinds in the first and second example. - Two examples are US 2011/0077633 A1 and GB 2 403 148 A. Elaborations are made to provide a better coupling factor to the OUS, for an acceptably wide range of OUS permittivities. However, a field pattern of the coaxial TEM type or the related circular TM₀₁ type at the OUS remains, resulting in a dominating nearfield non-propagation.
   All antenna systems referred to so far are nearfield and suitable for ablation and other spot heating purposes, but not for multiple antenna signal transmission and measurements through an OUS for microwave tomographic purposes. An advantage is, however, that there is only a weak peripheral electric field perpendicularly to the OUS surface outside the applicator, which would otherwise cause microwave surface waves.
4. Patch antennas. - A typical example is described in the paper A Comparison of a Wide-Slot and a Stacked Patch Antenna for the Purpose of Breast Cancer Detection in IEEE TRANSACTIONS ON ANTENNAS AND PROPAGATION, VOL. 58, NO. 3, MARCH2010. These are better in terms of frequency bandwidth but suffer from impedance matching problems necessitating their immersion in an external liquid (bolus) with comparable permittivity to that of the tissue. Boluses are intended for providing impedance matching improvement and "geometric control" of the overall OUS, as well as - by its dielectric losses - a reduction of disturbing stray propagation of the microwaves at and around the OUS. However, the bolus must then be in very close contact with the OUS, which may cause patient discomfort and also complicate the examination procedure.
5. Vivaldi antennas. - A typical state-of-the-art example is described in the paper Balanced Antipodal Vivaldi Antenna With Dielectric Director for Near-Field Microwave Imaging in IEEE TRANSACTIONS ON ANTENNAS AND PROPAGATION, VOL.58, NO. 7, JULY 2010. One can say that vivaldi antennas are developments based on so-called E-plane ridged flared horn antennas for microwave communication. An E-plane horn extends (flares) in only the direction of the narrow walls in a rectangular TE₁₀ waveguide. Vivaldi antennas are inherently frequency broadband (e.g. in the literature example from about 1 to 12 GHz) which makes them suitable for use with pulsing techniques, as in common radar systems. They may also be designed to have a favorable beam width, which is a very important performance factor. However, the electromechanical design is complicated and requires very tight tolerances, resulting in quite expensive antennas. Additionally, their behavior is modified by a microwave-absorbing OUS nearby.
6. Endfired ridged TE₁₀ waveguides as applicators for hyperthermia treatment. - An example is in US 4,282,887. The whole waveguide cross section is filled with a homogeneous high permittivity dielectric (water in this case). A combination of this and the ridged design results in a much reduced critical frequency than with no ridges and no filling. This makes it possible to employ a frequency lower than microwaves with an applicator of suitable end opening dimensions for treatment of quite large body parts such as thighs. The use of water having a permittivity higher than that of the human tissue can be employed to create a resonant system which increases the energy efficiency. The low frequency has the advantage of a deeper penetration depth of the propagation into the OUS having a permittivity comparable to that of the waveguide filling. There is, however, the disadvantage of a need for close contacting between the waveguide antenna opening and the OUS surface, since the wave emanating from the antenna is highly evanescent in this low permittivity gap of air, hair or fat tissues.

A typical limitation in situations like the above is, however, that the OUS is openly accessible from several directions, and has such a content of polar substances, such as water, that its permittivity is high. At the same time, the OUS may be surrounded by substances having much lower or no water contents, such as skull bones. This leads to challenges with respect to the coupling of electromagnetic fields into and out from the OUS.

Articles PETROVIC N ET AL: "Antenna applicator design for microwave imaging of the interior of human breasts",JOURNAL OF PHYSICS D: APPLIED PHYSICS, INSTITUTE OF PHYSICS PUBLISHING LTD, GB, vol. 47, no. 38, 28 August 2014 (2014-08-28), page 385401, ISSN: 0022-3727, DOI: 10.1088/0022-3727/47/38/385401 and MOBASHSHER AHMED TOAHA ET AL: "Microwave System to Detect Traumatic Brain Injuries Using Compact Unidirectional Antenna and Wideband Transceiver With Verification on Realistic Head Phantom",IEEE TRANSACTIONS ON MICROWAVE THEORY AND TECHNIQUES, IEEE SERVICE CENTER, PISCATAWAY, NJ, US, vol. 62, no. 9, 1 September 2014 (2014-09-01), pages 1826-1836,ISSN: 0018-9480, DOI: 10.1109/ TMTT.2014.2342669 and document US5841288 anticipate systems for detecting dielectric irregularities using electromagnetic emitter arrangements. Documents US 6160525 and US2015103859 and article TWIG YGAL ET AL: "Sensitive surface loop-gap microresonators for electron spin resonance",REVIEW OF SCIENTIFIC INSTRUMENTS, AIP, MELVILLE, NY, US, vol. 81, no. 10, 21 October 2010 (2010-10-21), pages 104703-104703,ISSN: 0034-6748, DOI: 10.1063/1.3488365 anticipate electromagnetic emitter arrangements as non-closed loops.

### Summary

An object of the present invention is to provide a system, and components thereof, for localization and quantification/characterization of inhomogeneities (deviations of the electromagnetic properties) in an OUS, in particular human breasts or heads.

The present invention is based on a number of insights about prior art systems and the associated limitations thereof.

Typically, boluses are used for enhancing the coupling of signals. As generally known by a person of ordinary skill in the art, a bolus is a reasonably tissue equivalent material placed on the body part, or OUS, to control the coupling of microwaves. A bolus in this context can, for example, be a thin-walled plastic bag filled with liquid and placed in close contact with the OUS. When boluses are used, the antennas can be located some distance away from the OUS in order to avoid the complicated microwave nearfield behavior of antennas at a dielectric boundary, and also to strongly reduce the creation of surface waves around the OUS periphery. With a sufficiently energy-absorbing bolus of sufficient size, simple and small monopole antennas can be utilized. However, boluses are awkward to use. They add weight and may cause contamination and cleaning issues. When the OUS can be and is submerged in the bolus liquid, overall systems become large and the need for pumping and purification becomes too complicated for normal clinical use.

Without a bolus, however, a first problem is how to avoid the generation of surface waves by the transmitting antenna. Surface waves propagate around a rounded OUS such as a breast or head with an attenuation very much less than that across the lossy OUS straight between the transmitting and receiving antennas. If the receiving antennas are similar to the transmitting antenna, the former will also pick up surface waves. Typically, damping devices or materials such as absorbing ferrites can be used for largely eliminating the surface waves, but the overall system still becomes sensitive to the precise contacting of the antenna to the OUS and the microwave properties of the OUS surface region.

Furthermore, wave propagation inside the OUS is of course damped by its microwave lossiness. Since also the receiving antenna is non-intrusive and is supposed not to significantly disturb the incoming signal to be received, the magnetic wall effect will cause problems. This effect is basically a total reflection effect similar to that in optics, and occurs when the permittivity outside the object is significantly lower than that within the object. Since the equivalent refractive index of typical human tissue is about 7 (the square root of the permittivity), the magnetic wall effect becomes very significant. Since the magnetic field of the magnetic wall effect will have a minimum at the boundary and is continuous with non-magnetic objects, the field impedance will be very high just outside the OUS surface. This will result in a poor performance of propagation-receiving antennas, i.e. structures intended to receive wave energy which is propagating.

As explained above, another factor for the invention is the so-called magnetic wall effect. Most of the wave energy will thus remain inside, by reflections at the boundary. With a high real permittivity this effect will be very strong. The result with frequencies according to the present invention will be an internal resonance-like field pattern if the loss factor is sufficiently low. If not, other related wave phenomena will also occur, such as arch-trapped (sometimes called creeping) waves along the boundary inside.

According to the invention, the emitter comprises a non-closed loop (metal wire or a metal tube/cylinder), configured as a cylinder having a slit through its wall along its axis and located close to the OUS and with its axis perpendicular to the OUS surface. The loop can be a part in an approximately resonant circuit by an added capacitor at its end opening or at the other end of a conductor pair. The ends with a capacitor can be shielded by conventional means, so that only a very weak electric field occurs from it at the OUS surface; this field is additionally reflected away from the OUS due to its high permittivity. The emitter has thus a spatially separated inductive part (the loop), resulting in only a dominant axial magnetic field entering the OUS. Since the OUS is non-magnetic, there are no damping or energy reflection effects at the OUS surface or just inside it. Due to Faraday's law, a circular "eddy current" path is generated inside the OUS, resulting in a corresponding circumferential electric field. This transverse combination of magnetic and electric field then propagates axially away from the emitter, then to become disturbed by any OUS dielectric inhomogeneity and reflected by the OUS boundary.

In embodiments of the invention, this method results in a propagating wave only inside the OUS, and this leads to the primary induced electric field just inside the OUS boundary becoming parallel to it. The consequence is that external surface waves are not excited at all.

The electromagnetic energy is, according to the present invention, applied by en emitting structure which is very different from the receiving structures. The emitter is a loop or cylinder producing a magnetic field directed radially inwards into the OUS. The receiver comprises resonant probes being selectively sensitive to the radially outwards-directed electric field emanating from the diffraction resulting from the internal dielectric property irregularity. They are thus designed as E-field probes rather than antennas in the conventional meaning. The receiving structures need to be small, which is accomplished by the resonant function being established in a high-permittivity ceramic structure.

The physical system according to the invention thus comprises an emitting device and a plurality of receiving devices. The method of extracting relevant data from the system, i.e. the procedures of use and data presentation, is not part of the invention.

Furthermore, waves diffracted by the inhomogeneities and by the OUS are primarily electric fields, since the OUS is non-magnetic, and become polarized perpendicularly to the direction of the primarily induced and stronger electric field, as well as to the primary magnetic field.

The suitable electromagnetic (microwave) frequency used depends on the physical size of the OUS, the required spatial resolution of the system in relation to the expected extent of the diffracting inhomogeneities, and the attenuation limitation of the wave propagation in the OUS. It is typically favorable to employ more than one operating frequency of the system.

The choice of operating frequency will typically be a compromise between the wave penetration depth (which is shorter for higher frequencies), the detectability of small inhomogeneities (which is better for higher frequencies), and imperfections of the loop design including difficulties to provide a quasistatic magnetic field undisturbed by tendencies for an electric field in it also to be created. The operating frequency is preferably 0.5 - 2.0 GHz, more preferably 0.8 - 1.2 GHz. An operating frequency of about 0.9 GHz has proven particularly useful for brain hemorrhage studies.

In embodiments of the invention, the loop (wire or tube) is spatially separated and shielded from the capacitive and energizing parts. This is since external electric fields may cause disturbing OUS surface waves, and the energy transfer efficiency from the magnetic field of the loop to the induced electric field in the OUS is not high.

Even though a property of the system is that the magnetic and electric fields are separated, there may be a need for a particular type of feed line to the loop/tube emitter. One property of the feed line can be expressed as its characteristic impedance, as if it were a transmission line. In embodiments of the present invention, a similar property is instead the related one that there should preferably be a minimum space between the two conductors of the feed line, where stray fields are created. One alternative is then to use a Litz wire, in which the individual strands of the core are very close and the core cross section can be distorted. Another alternative is to use a microstrip line with a very small distance between the opposing surfaces (however, the use of a microstrip line may be perceived as less flexible). The source should preferably be a balanced (i.e. equal conductors) current source providing the required current in the loop/tube, and be designed with a very low equivalent series impedance. According to the invention, the characteristic impedance of the feed line to the loop/tube is smaller than 20 Ohm, preferably below 10 Ohm and most preferably only 1-5 Ohm.

According to the present invention, the receivers used for picking up diffracted electromagnetic fields from the OUS are not antennas, but probes. They are designed for being sensitive to only the radially outwards-directed electric field. Such fields are basically created only by the internal dielectric irregularities in the OUS.

For example, spring-loaded multiple receiving probes are located around a single emitting loop and the received signals can directly, or after some relatively simple and very fast real-time processing, be directly visualized on e.g. a computer screen. By moving the assembly of loop and probes over the OUS, improvements in the analysis and finding of the size and location of the diffracting inhomogeneity in the OUS can be achieved.

### Brief description of the drawings

The geometrical definitions and the features of the present invention are illustrated on the following appended drawings, on which:
Figure 1 schematically shows components and fields in cross-section of a 150 to 200 mm diameter spherical OUS;
Figure 2 schematically shows the situation with two loops located along the periphery of the OUS, at some distance from each other and with their respective axes pointing at the center region of the OUS;
Figure 3 schematically shows a cross-section at the axis of a rotationally symmetric receiving probe;
Figures 4 and 5 schematically show momentary maximal resonant electric fields in the receiving probe; and
Figures 6a-d schematically show a part of an embodiment for contacting a human head;
Figure 7 shows an example using a plurality of loop emitters; and
Figure 8 schematically shows an embodiment of a loop emitter in the form of a tube/cylinder.

### Detailed description

The further embodiments of a system according to the present invention are now described, with reference to the figures and the above description of them.

Although the present invention will find various applications, it is envisaged that it will prove particularly useful for localizing and detecting/characterizing inhomogeneities in the human head. As briefly mentioned above, a human head cannot in any practical manner be surrounded by a sufficiently voluminous bolus or be submerged into a microwave absorbing liquid. This leads to a situation where there are good conditions for creation of surface waves, since antennas cannot be in contact with the high permittivity brain matter of the human head. In addition to the hair and skin layer, there are two types of skull bones with a total thickness of approximately 7 mm and an average ε' ≈ 16 and equivalent σ ≈ 0.25 Sm⁻¹ of the cortical and cancellous bone, at 1 GHz. Inside the bone, there is predominantly white and grey matter with an average ε' ≈ 45 and σ ≈ 0.8 Sm⁻¹ at the same frequency. Blood provides a contrast by its ε' ≈ 61 and σ ≈ 1.6 Sm⁻¹ at 1 GHz.

In general, the emitter should generate as little electric field as possible, since any electric field will in principle be able to create surface waves. Hence, a separation of the magnetic field from the electric field results in a magnetic-only field in the loop emitter, and it is therefore fed by a feed line of very low impedance. Furthermore, the loop circumference should be sufficiently small in order for wave phenomena not to occur at the operating frequency or frequencies. By avoiding wave phenomena from the loop emitter, surface waves are effectively also avoided, and propagating fields are only generated inside the head where the permittivity is sufficiently high.

The propagating fields generated inside the head (in the brain) will be diffracted by (the periphery of) any dielectric inhomogeneity such as a hemorrhage. The diffracted/deflected field components are essentially parallel to the primary magnetic field. Without diffraction from an inhomogeneity, no field of such polarization is created. This diffracted/deflected electric field can then be separated out from the (stronger) circulating electric field using an E-field probe according to the invention.

### Properties and embodiments of the emitting parts

For establishing the best system conditions, the loop axis should point towards the centre region of the OUS, i.e. to the region of the origos of the osculating circles of the OUS at the loop location. This will result in the most even path of the induced electric field (and by that current) in the OUS. An advantage of embodiments of the invention is that the distance between the loop and the nearest OUS surface is not sensitive; any induced electric field outside the OUS due to the action of Faraday's law will not create any surface waves, and non-quasistatic fields are eliminated by the size of the loop in relation to the free-space wavelength corresponding to the operating frequency. According to the present invention, the loop has a circumference that is sufficiently small in order to eliminate radiating fields. More particularly, the circumference of the loop is less than the free-space wavelength corresponding to the operating frequency of the feeding current. By free-space wavelength, it is meant the wavelength corresponding to the frequency according to the relation that wavelength times frequency always equals the propagation speed (e.g. at an operating frequency of 1 GHz, the free-space wavelength is 30 cm).

The combination of the essentially rotationally symmetric and mutually perpendicular magnetic and electric fields create an electromagnetic propagation in the OUS radially away from the loop axis, then to become disturbed by any OUS dielectric inhomogeneity and reflected by the OUS boundary. The disturbing irregularity creates a diffraction effect which results in main diffracted electric field components perpendicular to the direction of the primarily induced electric field, then mainly emanating in the locations of the irregularity edges perpendicular to the direction of the primarily induced electric field and with 180° phase difference at those edges.

A further and particular property of the invention is that the method of excitation results in a propagating wave only inside the OUS, and this leads to the induced electric field just inside the OUS boundary becoming parallel to it. The consequence is that external surface waves are not excited at all.

As mentioned above, the choice of operating frequency has to be a compromise between the wave penetration depth (which is shorter for higher frequencies), the detectability of small inhomogeneities (which is better for higher frequencies), and imperfections of the loop design including difficulties to separate the magnetic from the electric fields at it (this becomes more difficult for higher frequencies, and will contribute to possible creation of surface waves at the OUS).

Although the loop may be embodied as a loop of thin-wire design, a loop in the form of a circular tube or cylinder provides additional advantages. For example, such additional advantages may include:
- an improved structure of the magnetic field, as in a solenoid;
- moving the upward magnetic field away from the bottom end of the tube, thereby reducing the coupling to the OUS and also allowing an improved absorption by an object with magnetic losses positioned in the regions of the far end of the tube, which will further reduce spurious/unwanted electric and magnetic field components;
- allowing a half height feedpoint of the tube, resulting in a more symmetric magnetic field.

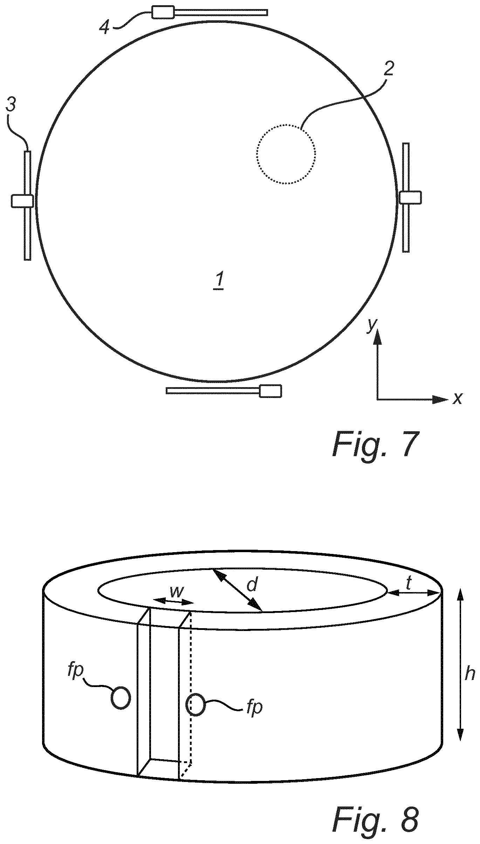

A schematic illustration of such embodiment of the loop emitter in the form of a cylinder/tube is shown in Figure 8, not drawn to scale.

As an example, for an operating frequency of 880 MHz, the inner diameter d of the tube may be 36 mm. The material thickness *t* may be 1.0 - 2.5 mm, and the tube may have a height *h* of 12 mm. Feeding of the tube may advantageously be made at a feedpoint *fp* at half its height. The slit width *w* of the tube (i.e. the non-closed loop) may be 0.5 - 2.0 mm for the operating frequencies of interest in embodiments of the present invention. The slit is preferably filled with some solid dielectric material for structural integrity and provided with an inner metal shield (omitted from Figure 8 for clarity) located along and inside the slit and having a width only slightly less than the slit. It is also conceivable to use an absorbing material for the shield rather than metal.

In order to reduce unwanted stray fields emanating at the tube/cylinder, it is preferred to in essence surround the tube/cylinder by a material having magnetic losses, and also conductive losses. This is preferably made around the outer circumference and, at some distance away from the end of the tube/cylinder, also near and across the opening distal (away from) from the OUS. The function of the surrounding material around the outer circumference of the tube/cylinder is to absorb all types of fields, while the function at the opening distal from the OUS is to reduce the B field in locations outside the zone between the tube/cylinder and the OUS. The lossy, or absorbing, material is suitably a ferrite with appropriate properties, and the specific selection can be made by the skilled person having read and understood the present disclosure. Optimization of the suppressing functions can be made by experiment, since the geometries of the applied material depends to some extent on the combination of magnetic losses and permittivity of the material. Examples of useful materials are flexible ferrite rubber gaskets or tapes, and thicker sintered bodies.

The basic function of the loop emitter (tube/cylinder) is to emit a quasistatic magnetic field only, but it may prove very hard to avoid generation also of electric fields. Such electric fields are easily guided by metal objects such as the feed lines and other cabling in the system. Good shielding may therefore be required in some embodiments, and can be achieved using conventional means, such as snap-on ferrites for cables or axial ferrite beads.

As mentioned above, with respect to the feeding of the loop emitter (tube/cylinder), the cabling should have low characteristic impedance, since this means that the electric field in the feed line is reduced compared with the surrounding magnetic field at the feed line. For example, it may therefore not be sufficient to use twisted Litz wire or microstrip for the feed line; in such cases shielding by an outer metal tube can be used. Similarly, a high-quality shielding is preferably provided also for the low output impedance amplifier/transformer that is used for feeding the loop emitter (tube/cylinder) via the feed lines.

Regions with poor detectability are unavoidable, partly because penetration depth effects resulting in poorer amplitude of the diffracted field by the OUS, and partly because the particular overall mode pattern in the OUS may be such that the diffraction amplitude from the inhomogeneity is weakened in relative terms. The remedy may include the use of
- different (i.e. moveable) emitter positions, then with fixed relative positions of the receiving probes;
- multiple emitters fed with the same or different phase but at the same frequency and being positioned such that magnetic field concentrations are obtained deeper into the OUS than by a single loop emitter;
- using more than one operating frequency, wherein each frequency has a corresponding receiving probe.

### Properties and embodiments of the receiver

There are several kinds of prior art antennas described in the literature for the general purposes of analysis of the internal properties of OUS:es by electromagnetic means. All are antennas in the sense that they are equally designed as transmitters and receivers, or as nearfield applicators for material dielectric property measurements. Examples of antennas described for microwave medical investigations of internal inhomogeneities in breasts and heads are patch antennas, Vivaldi antennas and endfire ridged TE₁₀ waveguide antennas, as mentioned in the Background section above. It is to be noted that these antennas are basically designed for sending and receiving propagating wave energy.

However, the probes according to the present invention are not designed for receiving energy (i.e. simultaneous electric and magnetic energy characterizing propagation), but specifically for discriminately sensing a particularly directed non-propagating evanescent electric field.

For such particular fields to become significant and be directly related to the inhomogeneity of the OUS, there is a need for the magnetic wall effect to exist at the external boundary of the OUS. In particular, this effect has to be so well developed that the field impedance of the evanescent field emanated from this surface is high. A normal consequence and limitation of the functionality of the receiver is thus that there is an internally diffracted electric field component perpendicular to the external surface of the OUS. This occurs in curved OUSs energized by a single loop with its axis directed to the OUS centre as previously described. Another way of obtaining the magnetic wall effect in e.g. an OUS with flat external surface is by using a declined loop or more than one loop.

The particular electric field probes of the invention are specifically designed for the property of being sensitive to largely only the external electric field in their axial direction, and not any electric fields parallel to the OUS surface. This is accomplished by using a circular TM₀₁ₚ mode resonant probe with a high permittivity filling and having a frustum conical end. The high permittivity filling has a dielectric constant about equal to or higher than that of the OUS, e.g. about 70. The resulting miniaturization is advantageous not only with regard to an improved spatial resolution when moved over the OUS, but also due to the OUS surface-parallel electric fields of opposite polarity at the probe opening becoming close, resulting in a much reduced sensitivity to the essentially constant external electric OUS surface-parallel field component over the probe receiving area, as induced by the loop emitter. This selectivity property is further enhanced by the frustum conical end part of the probe, which spatially raises the sensitivity region for the OUS surface-parallel electric field component above the OUS surface.

A further embodiment, not being part of the invention, is to design the receiving probe to have more than one resonant frequency and to employ that together with a variable frequency source. In particular, the circular TM₀₁₁ and TM₀₁₂ (open at both axial ends) modes can be employed. Such an example is shown in the description of the drawings. Approximately, these modes have resonant frequencies somewhat less than two and three times higher than the lowest frequency, with a simple probe design.

In order to further reduce the influence of stray fields that may exist outside the OUS surface, some embodiments will benefit from using a ferrite ring (or plate with a central opening) around the frustum end of the probe in contact with the metallization. Stray fields will then be suppressed by the ferrite, thus increasing the signal-to-noise ratio of the probe for detecting electric fields coming from the inside of the OUS.

Preferably, probes are positioned within ±90° from the emitter (loop) at 0° in order to obtain reasonable detection of inhomogeneities.

### Additional embodiments of the loop emitter and probe

The need for as far as possible eliminating surface waves at the OUS can be achieved in part by using longer free-space wavelengths than with the state-of-the-art electromagnetic applicators. Surface waves become very strong - what is labeled externally resonant - for an OUS circumference of about one or two free space wavelengths. For example 300 MHz, constituting the commonly defined lowest microwave frequency and having 1 meter free space wavelength, can in principle be used with e.g. a typical human head with 60 cm circumference, but will provide a very unsatisfactory spatial resolution. The only state-of-the-art remedy for higher frequencies is to use boluses, typically in combination with additional energy-absorbing means such as ferrite bodies.

It is an aim to create a propagating, albeit damped, wave inside the OUS. With a typical tissue real permittivity ("dielectric constant") ε of 30 or higher, the interior wavelength at e.g. 300 MHz becomes about 180 mm or less, since the linear size reduction factor then becomes the square root of the permittivity ε. At least a half wavelength in the OUS is then possible. This leads to the creation of an internal propagating mode, of course depending on the excitation by the emitter as well as the field absorption by losses in the OUS. As an example, white tissue in the brain has ε about 45 and equivalent conductivity (including the Debye relaxation losses) of about 0,5 S/m, corresponding to a loss factor about 30 at 300 MHz. This results in a plane wave penetration depth (1/e of the power density remaining below that) of about 37 mm.

More particularly, the use of an emitter in the form of a non-closed loop according to the present invention aims to provide a quasistatic magnetic field outside the OUS with an as small accompanying electric field as possible. It is also an aim to avoid the generation of wave propagation from the loop to as large extent as possible. This requires a loop length that is smaller than the free-space wavelength corresponding to the operating frequency (i.e. the frequency of the alternating current flowing through the coil). For a coil in the form of a thin loop, resonant conditions occur for a circumference to wavelength ratio of 1.10, while about 10% of the resonant field remains for a ratio of 0.75 and only 1% remains for a ratio of 0.50.

There will, however, inevitably be an electric field created at the loop opening where the current excitation is located. From a perspective of characteristic impedance, the design must thus be such that this is sufficiently low. Additional measures for reducing the electric field may include one or more of:
- using twisted leads to the gap, which will lead to the electric field at and around these twisted leads to be lowered due to the spatial direction variations occurring much more often than the distance corresponding to the wavelength of the operating frequency;
- using a dielectric in the loop gap, since this will lower the impedance thereof;
- using a metal shielding in the loop just inside the gap, further reducing the electric field in the magnetically active cross section of the loop.

An advantage of the invention is that the electrodynamic design in many cases allows immediate use of the received signals without any time-consuming statistical or mathematical post-processing. Suitable ways of such presentation of the measured data are presented in the section on signal processing and presentation.

A further advantage of the invention is related to no bolus being needed, and the immediate signal use. These factors allow a lightweight design of the assembly with a central loop emitter and a plurality of springloaded receiving probes around it to be manually moved over the OUS while observing the output signals. By this, a very much improved spatial sensitivity is obtained.

A further advantage is related to the use of the particular properties of the diffracted electric field from an internal inhomogeneity in the OUS. With the main direction of the induced electric field by the source magnetic field being rotational around the loop axis, the resulting diffracted perpendicularly (outwards) directed electric field will be strongest near the irregularity edges where the induced electric field is perpendicular to the edge. The diffracted field will have opposite phase at the opposite sides of these edges. By introducing electronic circuitry into the system that records the phase of the received signals by adjacent probes, an enhancement of the resolution of the irregularity is obtained.

As is evident from the foregoing, the possible uses of the present invention are not limited to the specific medical examples presented here. Provided the basic limitations of frequency versus size of the loop/tube and the OUS irregularities are kept in mind, the attenuation of the electromagnetic energy in the OUS and the need for access for measurements are fulfilled, a number of other applications both in industry and for medicine are viable. Such examples are detection of foreign objects in food, wood and chemical processing and characterization of internal properties in e.g. concrete and wood.

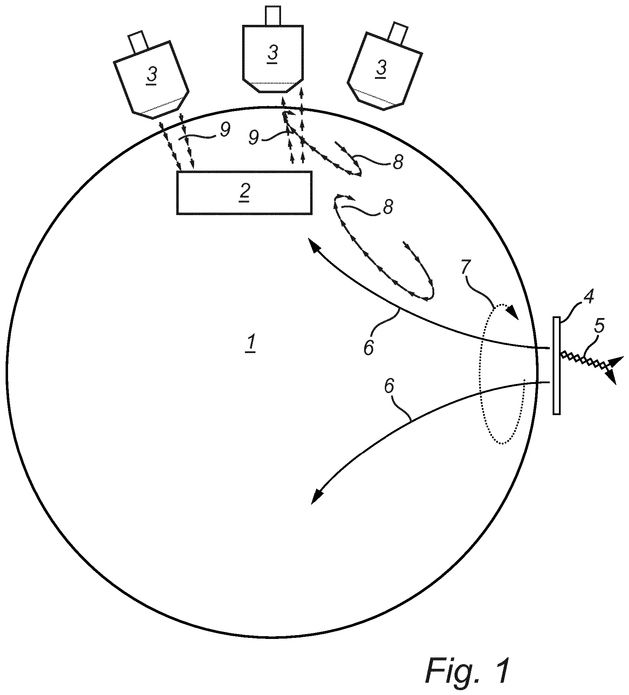

Figure 1 schematically shows components and fields at and in a cross section of a 150 to 200 mm diameter spherical OUS 1 with a puck-shaped dielectric inhomogeneity 2 having a higher permittivity. The circular, typically single turn loop 4 is fed by a twisted conductor pair 5 for reducing any extraneous fields, but the shielded box in which the leads are connected to the resonance capacitor and the resonant frequency generator are not shown, neither is any ferrimagnetic absorbing body of the magnetic fields on the back side of the loop. Two resulting magnetic field lines 6 in the OUS 1 are shown. They create an initial circulating electric field 7. This field and the magnetic field are successively transformed into a propagating energy which is very schematically shown by its electric field lines 8. It is to be noted that a propagation direction both following the OUS 1 surface region as well as mode patterns are created, as determined by the internal wavelength in the OUS, by the original propagation direction radially outwards from the loop axis, and the wave trapping at the OUS boundary by the magnetic wall effect. The diffraction by the inhomogeneity 2 creates electric fields 9, perpendicularly to the direction of the impinging electric field 8. Both the surface-parallel and surface-perpendicular fields thus exist in the receiving regions of the probes 3. Both field types are quite strongly evanescent outside the OUS boundary, largely due to the magnetic wall effect resulting in a quite absent magnetic field there. It is to be noted that whereas the surface-parallel electric field is continuous across the boundary, the perpendicular is amplified due to the requirement on continuity of the displacement (*D* field) vector.

Figure 2 schematically shows the situation with two loops 4 located along the periphery, at some distance from each other and with their axes pointing generally at the center region of the OUS 1. The magnetic field lines 6 now combine vectorially into field lines 6A as shown schematically, and which have a higher strength inside the OUS 1, the region of the shown rotating electric field 7. This main induced electric field 7 is thus a major contributing source to the propagating energy which goes in the direction as shown by the smaller electric field loops 8. The magnetic wall effect is enhanced, as is also the detectability of more deeply situated dielectric inhomogeneities in the OUS 1.

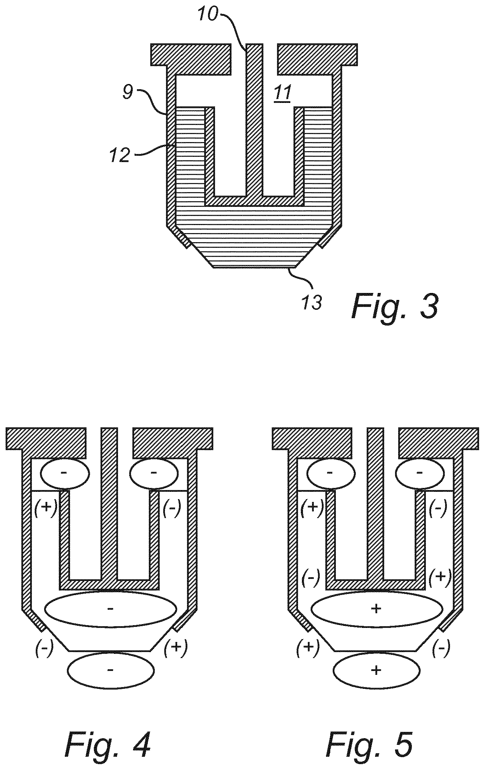

Figure 3 shows the cross section at the axis of the rotationally symmetric receiving probe 3. Its external metallic body 9 has an inner coaxial conductor 10, which is extended into a cup-shaped crevice in the ceramic body 12, so that an airspace 11 is created. This acts as an open end for the resonance field in the partially hollow ceramic body 12, and also for coupling factor adaptation. The high permittivity ceramic part 12 has a lower frustum conical open part which is only partially covered by the metallization 9. The open reception area 13 remains, for sensing the external axially directed electric field. As to the overall dimensions of the receiving probe 3, a diameter of the ceramic body of about 20 mm provides a TM₀₁₁ resonant frequency of about 1 GHz, with a ceramic permittivity of about 70. Preferably, the external metallic body is provided in the form of a multilayer deposition, comprising an inner adhesive layer (e.g. molybdenum) and an outer protective layer (e.g. silver alloy).

Figure 4 schematically shows the momentaneous maximal resonant electric fields in and around the ceramic body shown in Figure 3. The + and - signs in closed curves are the axial field, and signs in parentheses are the radial fields. Since both axial ends of the ceramic part are open, the mode has half a vertical wavelength and is thus of the open-ended circular TM₀₁₁ type. A particular feature is that the vertical field becomes stronger in the air below the opening due to the electric displacement (*D*) vector continuity. Another feature is that the radial field has different directions at the opening, which largely eliminates the probe sensitivity to any external constant such field. This discrimination is enhanced by the frustum conical end shape embodiment bringing the (+) and (-) areas closer, and the embodiment of the metallization not going all the way to the end.

Figure 5 is analogous to Figure 4, and shows the electric fields for the open-ended circular TM₀₁₂ type resonance. The fact that resonant modes with different *p* indices can be excited in the same applicator is a useful advantage. As a matter of fact, also the TM₀₁₃ mode can easily be excited in the same probe system, and also the TM₀₁₀ mode having a significantly lower resonant frequency can be excited with a shorter axial length of the ceramic part.

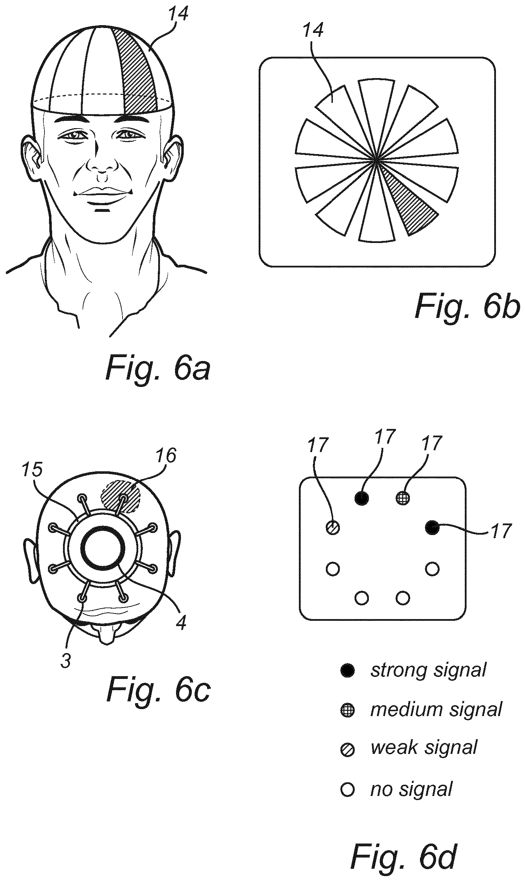

Figures 6a-d show some features and embodiments of that part of the overall system according to the invention which is contacting a human head with the intent of finding and characterizing internal inhomogeneities such as a hemorrhage 16 in the brain. One emitting loop 4, a support ring 15 above, for the springs 14 and several receiving probes 3 are shown. This may be a hand-held unit, connected to a feeding circuit and receiving circuits. The measurement system is shown in an intended position when in use. All shielding and protecting mechanical structures are omitted from the figures, for clarity. The system connections are not shown. The head with the moveable springs 14 on the head is shown in Figure 6a. It is split into a number of sector-shaped springy parts shown flattened in Figure 6b, each being equipped with a probe 16 as shown in Figure 6c. Thus, the 3D conformal surface of the head is translated to a 2D planar representation of the measurement results. Distortion of the geometry is unavoidable but still gives an indication of the position of irregularities. The execution assures that all probe ends 13 are contacting the head surface as shown in Figure 6c where also the central loop emitter 4 of a unit 14 fixture ring 15 are shown. Now supposing an inner inhomogeneity 16 to be located as shown in Figure 6c, the probe outputs can schematically be shown by indicators 17 as shown in Figure 6d, after amplification and other signal processing known by anyone skilled in the art. This is a simplified alternative representation where the received signal intensity shows directly on a screen or even using diodes placed directly on the probes.

As described above, maximal signals will be obtained from probes closest to the rims of the inhomogeneity 16 as encountered by a circular electric field parallel to the loop. As a consequence, the relative signal strengths will be as indicated in Figure 6d, allowing determination of the projected location if the inhomogeneity 16 as well as its approximate extent. An extended signal processing system providing the relative phases of the two signals marked as strong in Figure 6d will result in opposite phase recordings in these locations of indication. This is a further embodiment of the invention which provides an improved discrimination of the inhomogeneity 16 characteristics, and also reduces the noise level in the reception system by added circuitry as evident to anyone skilled in the art.

### Signal processing and presentation

Some aspects are already addressed above. This phase difference effect can be made visible through color coding or the use of more light sources than shown in Figure 6d. The obvious representation of the measurement results giving a 3D image on the screen is not shown in any of the Figures but is a possible way of representing the shape and location of the irregularity.

Another advantageous possibility offered by the small size and weight of the assembly is that the irregularity can be further defined by moving the center of the measurement system around the area of interest. This feature is of great practical importance and is available by the practically immediate signal presentation in geometric terms. Not only the likelihood of finding the inhomogeneity but also an improved resolution and sensitivity result. This feature can of course also be used to reduce the number of probes of the system. Finally, there will be no need for the sensors to cover more than a part of a rounded OUS when being a breast, skull or cylindrical object when the hand-held part shown in Figure 6 is manually moved under direct visual observation of the representation of the measured data.

After signal processing, the measurement results can be shown on a screen or directly on the hand-held unit. This can be moved on the OUS to further investigate areas of interest and to expand the measurement area. An IMU (Inertial Measurement Unit) can be included in the hand-held unit in order to keep track of the movement and be able to translate to the right positions in the represented object image, if shown on a screen. The IMU is calibrated before each measurement session by placing the hand-held unit in a pre-defined position on the object. The signal processing includes amplification and appropriate filtering of the received signals before the amplitude and the phase shift of each probe are calculated. Several different versions of the system can be created: simple systems showing received, unprocessed signal strength directly on the hand-held unit, or more advanced systems all the way up to IMU controlled movement and 3D computer screen representation of the object, based on both amplitude and phase information and image processing.

The use of more than one operating frequency is advantageous, since "dead regions" of a fixed frequency field pattern in the OUS then typically disappears. It is then advantageous to design the signal processing and presentation systems for very quick back-and-forth frequency switching. This can of course be automated and included in the IMU system described above.

By using multiple loops, as schematically shown in Figure 7, several advantages can be obtained when these loops are excited by the same source in such a phase that two opposing loops provide a magnetic field in the same direction. There will then be a mutual coupling between the loops, resulting in the field lines being quite straight and of about the same strength in the whole space between the loops and limited by the loop opening areas. This will result in (a) the induced electric field to become more homogeneous (equal in strength and shape) in the space surrounding this across magnetic field zone; and (b) possibilities to detect deeper inhomogeneities than with a single loop emitter.

Another example may use two adjacent loops, e.g. two 90° displaced loops as shown in Figure 7. These loops, as well as suitable located E-field probes, then do not need to be moved if the strength and phase of the current fed to the loops are changed (typically periodically at a low frequency, or stepwise according to a predetermined scheme). These changes will result in differences in the signals received by the E-probes. An advantage of this is that any signal strength differences due to, for example, differences in head hair layer or fat layer thicknesses are eliminated, so that only signal changes caused by differences in diffraction by an internal inhomogeneity remain.

It may also be noted that by using three symmetrically located loops with axes in one plane and the common axis point in the middle will create a rotating H field when the three loops are fed with 120° phase difference (i.e. "three-phase"). An advantage of such a configuration is that the inhomogeneity will be "illuminated" from many directions so that the likelihood of detection increases.

### Conclusion

A system and method, as well as sub assemblies thereof, for detection of dielectric irregularities/inhomogeneities inside an object under study (OUS) be means of electromagnetic energy are disclosed. The system comprises a loop/cylinder emitter configured as a cylinder having a slit through its wall along its axis and to be located close to the OUS with its axis of symmetry directed towards the OUS. A feeding line feeds the emitter with an alternating current at an operating frequency to cause a magnetic field therein, which in turn will induce a propagating electromagnetic field in the OUS. In order to reduce propagating fields outside of the OUS, the circumference of the emitter is smaller than the free-space wavelength corresponding to the operating frequency, and the feeding line has a characteristic impedance that is smaller than 20 Ohm.

## Claims

1. An electromagnetic emitter arrangement, comprising
a metallic, non-closed loop (4) with an axis of symmetry, said non-closed loop (4) being configured as a cylinder having a slit through its wall along its axis and said loop (4) being configured to be located in the vicinity of an object under study, OUS, in such a way that the axis of symmetry is directed towards the OUS;
a feeding line (5) configured to feed the loop (4) with an alternating current at an operating frequency to cause a magnetic field in the loop (4);
wherein said loop (4) has a circumference that is smaller than a free-space wavelength corresponding to the operating frequency; and
said feeding line (5) has a characteristic impedance that is smaller than 20 Ohm.

2. The arrangement of claim 1, wherein said slit is filled with a dielectric material.

3. The arrangement of claim 2, further comprising a metallic strip on the inner surface of said dielectric material, said metallic strip preferably having a thickness of 0.5 - 5 mm.

4. The arrangement of any one of claims 1-3, wherein the circumference of the non-closed loop is smaller than 75% of said free-space wavelength, preferably about half of said free-space wavelength.

5. The arrangement of any one of claims 1-4, wherein said feeding line is coupled to an energy source via a balun.

6. The arrangement of any one of claims 1-5, wherein said feeding line comprises a Litz wire.

7. The arrangement of any one of claims 1-6, wherein said feeding line has a characteristic impedance that is smaller than 10 Ohm.

8. A system for detection of dielectric irregularities inside an object under study, OUS, by means of electromagnetic energy, the system comprising
an electromagnetic emitter arrangement according to any one of the preceding claims; and
a probe (3) configured to selectively receive an outwards-directed electric field component proximate to the OUS outer surface.

9. The system of claim 8, wherein the probe comprises an E-field probe that is configured to discriminate between surface-parallel and surface-perpendicular electric field components.

10. The system of claim 9, wherein the probe comprises a contacting end for contacting the OUS, said contacting end having a symmetrical frustum conical shape.

11. The system of any one of claims 8-10, wherein the operating frequency is 0.5 - 2.0 GHz, preferably 0.8 - 1.2 GHz.

## Patentansprüche

1. Elektromagnetische Emitteranordnung, umfassend eine metallische nicht geschlossene Schleife (4) mit einer Symmetrieachse, wobei die nicht geschlossene Schleife (4) als ein Zylinder mit einem Schlitz durch seine Wand entlang seiner Achse konfiguriert ist und die Schleife (4) konfiguriert ist, sich in der Nähe eines zu untersuchenden Objekts OUS zu befinden, sodass die Symmetrieachse in Richtung auf das OUS gerichtet ist;
eine Speiseleitung (5), die konfiguriert ist, die Schleife (4) mit einem Wechselstrom mit einer Betriebsfrequenz zu speisen, um ein Magnetfeld in der Schleife (4) zu erzeugen;
wobei die Schleife (4) einen Umfang aufweist, der kleiner ist als eine Freiraumwellenlänge, die der Betriebsfrequenz entspricht; und
die Speiseleitung (5) eine Kennimpedanz aufweist, die kleiner als 20 Ohm ist.

2. Anordnung nach Anspruch 1, wobei der Schlitz mit einem Dielektrikum gefüllt ist.

3. Anordnung nach Anspruch 2, ferner umfassend einen Metallstreifen auf der Innenfläche des Dielektrikums, wobei der Metallstreifen bevorzugt eine Dicke von 0,5 bis 5 mm aufweist.

4. Anordnung nach einem der Ansprüche 1 bis 3, wobei der Umfang der nicht geschlossenen Schleife kleiner als 75 % der Freiraumwellenlänge ist und bevorzugt ungefähr die Hälfte der Freiraumwellenlänge beträgt.

5. Anordnung nach einem der Ansprüche 1 bis 4, wobei die Speiseleitung über ein Symmetrierglied mit einer Energiequelle gekoppelt ist.

6. Anordnung nach einem der Ansprüche 1 bis 5, wobei die Speiseleitung einen Litzendraht umfasst.

7. Anordnung nach einem der Ansprüche 1 bis 6, wobei die Speiseleitung eine Kennimpedanz aufweist, die kleiner als 10 Ohm ist.

8. System zur Detektion dielektrischer Unregelmäßigkeiten innerhalb eines zu untersuchenden Objekts OUS mittels elektromagnetischer Energie, wobei das System eine elektromagnetische Emitteranordnung nach einem der vorstehenden Ansprüche umfasst; und
eine Sonde (3), die konfiguriert ist, eine nach außen gerichtete elektrische Feldkomponente in der Nähe der OUS-Außenfläche selektiv zu empfangen.

9. System nach Anspruch 8, wobei die Sonde eine E-Feldsonde umfasst, die konfiguriert ist, zwischen oberflächenparallelen und oberflächensenkrechten elektrischen Feldkomponenten zu unterscheiden.

10. System nach Anspruch 9, wobei die Sonde ein Kontaktende zum Kontaktieren des OUS aufweist und das Kontaktende eine symmetrische kegelstumpfförmige Form aufweist.

11. System nach einem der Ansprüche 8 bis 10, wobei die Betriebsfrequenz 0,5 bis 2,0 GHz und bevorzugt 0,8 bis 1,2 GHz beträgt.

## Revendications

1. Agencement d'émetteur électromagnétique comprenant une boucle métallique, non fermée (4) ayant un axe de symétrie, ladite boucle non fermée (4) étant conçue comme un cylindre ayant une fente en travers de sa paroi le long de son axe et ladite boucle (4) étant conçue pour être située à proximité d'un objet sous étude, OUS, d'une manière telle que l'axe de symétrie est dirigé en direction de l'OUS ;
une ligne d'alimentation (5) conçue pour alimenter la boucle (4) avec un courant alternatif à une fréquence de fonctionnement permettant de provoquer un champ magnétique dans la boucle (4) ;
dans lequel ladite boucle (4) a une circonférence qui est inférieure à une longueur d'onde en espace libre correspondant à la fréquence de fonctionnement ; et
ladite ligne d'alimentation (5) a une impédance caractéristique qui est inférieure à 20 Ohm.

2. Agencement selon la revendication 1, dans lequel ladite fente est remplie d'un matériau diélectrique.

3. Agencement selon la revendication 2, comprenant en outre une bande métallique située sur la surface intérieure du matériau diélectrique, ladite bande métallique ayant de préférence une épaisseur comprise entre 0,5 et 5 mm.

4. Agencement selon l'une quelconque des revendications 1 à 3, dans lequel la circonférence de la boucle non fermée est inférieure à 75 % de ladite longueur d'onde en espace libre, de préférence environ la moitié de ladite longueur d'onde en espace libre.

5. Agencement selon l'une quelconque des revendications 1 à 4, dans lequel ladite ligne d'alimentation est connectée à une source d'énergie par l'intermédiaire d'un symétriseur.

6. Agencement selon l'une quelconque des revendications 1 à 5, dans lequel ladite ligne d'alimentation comprend un câble de Litz.

7. Agencement selon l'une quelconque des revendications 1 à 6, dans lequel ladite ligne d'alimentation a une indépendance caractéristique qui est inférieure à 10 Ohm.

8. Système de détection d'irrégularités diélectriques à l'intérieur d'un objet sous étude, OUS, au moyen de l'énergie électromagnétique, le système comprenant
un agencement d'émetteurs électromagnétiques selon l'une quelconque des revendications précédentes ; et
une sonde (3) conçue pour recevoir sélectivement un composant de champ magnétique dirigé vers l'extérieur et située à proximité de la surface extérieure de l'OUS.

9. Système selon la revendication 8, dans lequel la sonde comprend une sonde de champ E qui est conçue pour discriminer entre des composants de champ électrique parallèle à la surface et des composants de champ électrique perpendiculaire à la surface.

10. Système selon la revendication 9, dans lequel la sonde comprend une extrémité de contact permettant de mettre en contact l'OUS, ladite extrémité de contact ayant une forme conique à tronc symétrique.

11. Système selon l'une quelconque des revendications 8 à 10, dans lequel la fréquence de fonctionnement est comprise entre 0,5 et 2,0 GHz, de préférence comprise entre 0,8 et 1,2 GHz.
